Europäisches Patentamt

European Patent Office

Office européen des brevets

(11) Publication number: **0 037 768**
**B1**

(12) **EUROPEAN PATENT SPECIFICATION**

(45) Date of publication of patent specification: **12.09.84**

(21) Application number: **81400479.2**

(22) Date of filing: **26.03.81**

(51) Int. Cl.³: **A 61 F 1/22**

(54) **Pivoting disc heart valve.**

(30) Priority: **27.03.80 US 134368**

(43) Date of publication of application:
**14.10.81 Bulletin 81/41**

(45) Publication of the grant of the patent:
**12.09.84 Bulletin 84/37**

(84) Designated Contracting States:
**DE FR GB SE**

(56) References cited:
**FR-A-2 322 580**
**US-A-3 825 957**
**US-A-4 021 863**

(73) Proprietor: **MEDTRONIC, INC.**
**3055 Old Highway Eight P.O. Box 1453**
**Minneapolis Minnesota 55440 (US)**

(72) Inventor: **Johnson, Keith M.**
**11708 Larch Street N.W.**
**Coon Rapids, Minnesota 55433 (US)**

(74) Representative: **Lavoix, Jean et al**
**c/o Cabinet Lavoix 2, Place D'Estienne D'Orves**
**F-75441 Paris Cedex 09 (FR)**

Courier Press, Leamington Spa, England.

## Description

The present invention relates generally to artificial body members, and more importantly, pertains to a pivoting disc heart valve.

The replacement of damaged or diseased natural heart valves with mechanical heart valves has become medically quite common. Of the various mechanical heart valve designs which have been proposed, pivoting disc valves of the type disclosed, for example, in U.S. Patents 3,825,957 and 4,021,863, have the significant feature of providing minimal interference to blood flow through the heart valve. The valves of the two above-mentioned patents each employ a pivoting disc having a center hole and a guide strut which extends through the center hole guiding the disc between its open and closed positions. The valves include a generally annular valve body having various inwardly directed projections arising from its inner surface where the projections act as pivot surfaces urging the disc to pivot between open and closed positions in response to cyclic blood pressure differentials across the valve. The manufacture of the valves such as those described in the above-mentioned patents is a meticulous process which involves machining and polishing the valve body and associated disc-supporting structure to exacting tolerances.

Heart valves should disturb blood flow a little as possible. The orifice of the heart valve should be as free as possible of internal projections disturbing the blood flow. It is particularly important from the standpoint of blood flow dynamics that the heart valve be as free as possible of obstructions immediately upstream from the orifice of the heart valve. On the other hand, in order to avoid interference with the heart valve operation from encroaching heart tissue downstream of the heart valve, the heart valve should further be as free as possible of physical structure extending downstream from the heart valve. Finally, the heart valve must be structurally strong, and be physically capable of reliable operation in opening and closing through millions of cycles.

In addition, it has been recognized in the past that the closure of heart valves causes an audible "clicking" sound which is distinctly unpleasant to the patient as well as those around him or her. In one prior art valve, to be discussed hereinafter, this clicking was eliminated or reduced by providing a combination of structural features which utilized the cushioning action of the blood itself. A stop shoulder having a flat surface contact interface area between the shoulder and the valve disc was provided and inflow pivot members also having surface contact interface areas between the pivot and the valve disc were also provided whereby, when the disc was closing the disc surface and the surface interface areas of the shoulder and pivot members would entrap or pinch blood cells and thereby cushion the closing of the disc. It has, however, been discovered that this solution to the clicking problem causes damage to the blood cells by the pinching action.

The pivoting disc heart valve of the present invention incorporates a pivot integrally onto a central guide strut. The guide strut provides for positive retention of a radiopaque occluder, and simplifies manufacture.

The present invention provides a pivoting disc heart valve including radially inward disc supporting projections and having physical structure stability.

According to one embodiment of the present invention, there is provided a heart valve for controlling the flow of blood which includes a valve base of a generally annular configuration defining a circular opening therethrough, the valve base having an outflow side and an inflow side and an interior wall, inflow pivot members extending inwardly from the wall into the opening along a chordal axis towards one another, the inflow pivot members being spaced towards the inflow side of the valve base, an outflow pivot member extending radially into the opening from the wall and perpendicular to the chordal axis of the inflow pivot members, the outflow member being on the same side of said opening as the chordal axis, a disc guide strut projecting generally inwardly from the wall towards the center of the valve base opening, the strut projecting from the wall from the side of the opening opposite the outflow pivot member, the strut defining an S-shaped curve extending diagonally upstream out of the valve base and curving back to pass axially through the center of the opening and then curving diagonally downstream of the valve base to terminate in a free end, a valve disc of circular shape having a diameter slightly smaller than said opening, the valve disc defining an axial orifice therethrough of a diameter slightly larger than the diameter of the guide strut, the valve disc being mounted in the valve base with the guide strut extending through the orifice and being positioned when in the closed mode position intermediate the inflow pivot members and the outflow pivot member, a valve disc stop shoulder extending inwardly from the wall on the side of the opening opposite the outflow pivot member, the inflow pivot members each having a first surface angled at about 70°—75° with respect to the plane of the valve disc when the disc is in the closed mode, said first surface of the inflow pivot members defining the maximum open mode position of the valve disc.

According to the invention, such a valve is characterized in that the valve disc stop shoulder has a rounded surface and presents a shoulder edge, the shoulder edge providing a line contact with the disc in the closed mode position of the disc, and in that the inflow pivot members each have a second surface angled at about 15°—20° with respect to the plane of said valve disc when the disc is in the closed mode, the first and second surfaces of the pivot members meeting to form pivot edges, the pivot edges providing line contacts with the disc in the closed mode position of the disc.

It has been discovered that the line contacts provided by the valve disc stop shoulder and the pivot edges of the inflow pivot members will reduce the blood cell damage caused by repeated closings of the valve disc while, when the inflow pivot members have the second surface angled at about 15°—20°, there will be adequate cushioning action of the disc to prevent clicking.

In the open position, the disc of the heart valve is centrally located within the valve orifice, thereby improving hemodynamic efficiency, reduced flow resistance, increased effective valve orifice area, and reduction of flow stasis on the outflow side of the disc.

One piece construction is achieved by machining a solid piece of metal into the final configuration with an absence of bends, welds or joints. This construction preserves the original molecular integrity of the metal thereby insuring long term structural integrity.

Other objects and many of the attendant advantages of this invention will be readily appreciated as the same becomes better understood by reference to the following detailed description when considered in connection with the accompanying drawings, in which like reference numerals designate like parts throughout the Figures thereof and wherein:

Fig. 1a is a plan view of a prior art heart valve with the disc partially illustrated for purposes of clarity in illustration and explanation;

Fig. 1b is a cross-sectional view taken along line 1b—1b of Fig. 1a;

Fig. 1c is a cross-sectional view taken along line 1c—1c of Fig. 1a;

Fig. 2a is a plan view of a heart valve of the present invention with the disc partially illustrated for purposes of clarity in the illustration;

Fig. 2b is a cross-sectional view taken along line 2b—2b of Fig. 2a; and

Fig. 2c is a cross-sectional view taken along line 2c—2c of Fig. 2a.

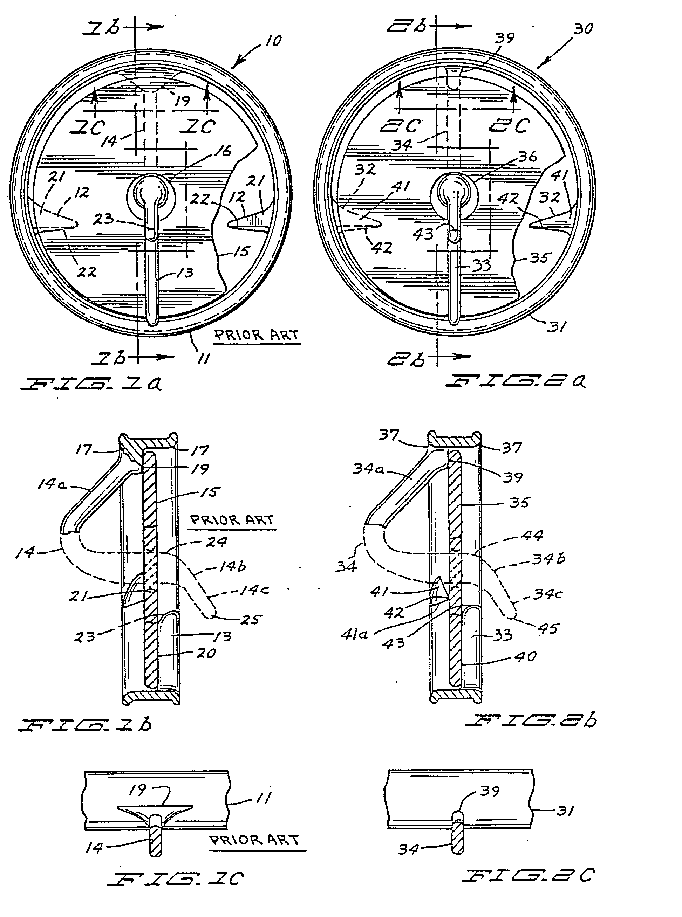

Fig. 1a, which illustrates a plan view of a prior art heart valve with the disc partially illustrated for purposes of clarity in illustration and explanation, shows a heart valve 10 having a valve housing member which includes an annular valve base 11 which provides support for a pair of opposing integral inflow pivots 12. Pivots 12 are identical to one another except for being mirror images and are positioned along a chordal axis of the interior periphery of the valve base 11. The outer edges of the valve base 11 are curved for providing a rim for holding a sewing ring. Extending transversely to the chordal axis of inflow pivots 12 and radially toward the center of annular member 11 is an outflow pivot 13 which cooperates with a disc guide strut 14 to guide a disc 15 during the opening and closing operation of the valve. Members 13 and the operational portions of 14 and the axis of the opening defined by the valve base 11 are in planar alignment with one another at right angles to the chord of pivots 12. The disc 15 includes a central opening 16. The disc 15 is made as thin as possible commensurate with needed strength so as to minimize the mass involved. The disc material may be Pyrolite, or other suitable material, and all peripheral edges as well as the internal edges of opening 16 are rounded. The outer diameter of disc 15 is slightly less than the diameter of the opening through the annular valve base 11. The opening 16 is of a larger diameter than the largest portion of valve guide strut 14. The valve base 11 is provided with outwardly bell-shaped openings toward opposite sides thereof as can be seen in the several figures. This curvature is indicated by numeral 17.

Fig. 1b, which illustrates a cross-sectional view taken along line 1a—1a, shows the valve housing 10 having the disc guide strut generally designated 14 extending inwardly from a peripheral inward edge. At the point of juncture of disc guide strut 14 with the valve housing, there is provided a shoulder 19 which acts as a stop for disc 15 as also illustrated in Fig. 1c, a cross-sectional view of Fig. 1a taken along line 1c—1c of Fig. 1a. Disc 15 is stopped on the other side of valve housing 10 to shoulder 19 by shoulder portions 21 of inflow pivots 12. The inflow pivots 12 extend inwardly into the inner portion of the valve base 11 along a chordal axis as shown and are integral with the ring 11 so as to provide structural integrity. Pivots 12 are provided with a rounded edge portion 22 which acts as the pivot point for the disc. Stop 19 and the shoulder 20 of outflow pivot 13 are spaced axially apart a distance slightly more than the thickness of the disc 15. Thus, when the disc is at the closed or rest position, it lies in a plane substantially perpendicular to the axis of the annular valve base with the shoulders 19 and 21 providing stop members. The disc guide strut 14 is shaped generally as an S. It projects downwardly from the interior wall or valve base 11 at a substantial angle and then, as it nears the axis of the valve base, makes an S-shaped curve to pass axially through the center of valve base 11. After passing through the central portions of valve base 11, it then turns again to terminate in a short section to complete the S-shape. More specifically, the disc guide strut at the point 14a that departs from the valve base rim has a generally oval or ellipsoidal cross-section until it passes through the first curve of the S. At this point, it thickens gradually as shown until the central portion 24 is round and of a diameter closely approximating the diameter of the opening 16 in disc 15. This is accomplished through a gradual tapering outwardly of the guide strut. The guide strut proceeds axially through the center of the valve base and is then gradually tapered again to a smaller dimension 14b which may be circular or of oval or ellipsoidal cross-section, and the second S-shaped portion 14c is produced which may be a straight line section over a short distance until the end of the guide strut is reached. This latter portion functions to restrain the disc 15 from disassociating free of the assembly and also permits, by its narrowed cross-section, movement of the valve disc 15 in a direction outwardly away from the valve base 11 to prevent less obstruction to flow of blood through the central orifice of the valve base. The outflow pivot 13, as already indicated, includes shoulder surface 20 perpendicular to the axis of the annular valve base. The cross-section of member 13 is an oval or ellipsoidal shape with the major axis in the direction of blood flow. The outflow pivot 13 extends a substantial distance into the interior of the opening in the valve base 11. The central internal surface 23 of member 13 is curved with a gradual radius to provide the pivot point for the valve disc 15 as it goes into the open position. The central-most edge of member 13 is sloped to provide an angle of about 70°—75° to the axis of the valve base axis, which angular surface combines with the inflow pivots 12 and determines the maximum ultimate angle that valve disc 15 will assume in the open position.

Fig. 1c, which illustrates a cross-sectional view along line 1c—1c of Fig. 1a, shows numerals which correspond to those elements previously described.

Fig. 2a, which illustrates a plan view of the heart valve of the present invention with the disc partially illustrated for purposes of clarity in illustration and explanation, shows a heart valve 30 having a valve housing member which includes an annular valve base 31 which provides support for a pair of opposing inflow pivots 32. The structure of the heart valve 30 of the present invention and the prior art heart valve 10 differ with respect to the shoulders 41 of the integral inflow pivots and the disc stop 39 also referred to as shoulder 39. Otherwise, the structure between the heart valve 30 of the present invention and the prior art heart valve 10 are identical not only in physical structure but in operation. The integral inflow pivots 32 differ by having an angular surface indicated as a shoulder 41. The angle of the shoulder 41 is best illustrated in Fig. 2b. The disc 35 is at an angle in the range of 15°—20° with respect to the surface of the shoulder in its closed position. The angle of the shoulder provides for line contact when the disc is closed as opposed to surface contact in the prior art. Since the remaining structure of the heart valve 30 is identical, discussion of the elements of the outflow pivot 33, disc guide strut 34, disc 35, central opening 36, outward bell-shaped opening of the housing 37, shoulder 40, rounded edge portion 42, curved end 43, central portion 44, and disc guide strut end 45 are not discussed, as the discussion for the identical elements of the prior art as applicable herein is not repeated for the purpose of brevity in the specification. The shoulder 39 in Fig. 2a is illustrated as being considerably smaller and also as being rounded in Fig. 2c thereby eliminating surface contact and providing for line contact with the surface of the disc 35 in its closed position.

Fig. 2b, which illustrates a cross-sectional view taken along line 2b—2b of Fig. 2a, where the numerals correspond to those elements previously described. Particularly, the figure shows the angled shoulder 41 at an angle about 15°—20° with respect to the disc 35 and the shoulder 39. The integral inflow pivot 32 shows line contact at pivot edge 42 with the disc 35, one of the inventive significant aspects and features of the present invention.

Fig. 2c, which illustrates a cross-sectional view taken along line 2c—2c of Fig. 2a, shows the rounded, shoulder 39 which provides for line contact with the disc 35, the other inventive significant aspect and feature of the present invention.

In the closed position, the plane of the valve disc 35 is parallel to the plane of the valve base 31 of the heart valve 30. In this position, the disc 35 rests on the line contact of pivot edges 42 of the two inflow pivots 32 and of the disc stop 39 of the disc guide strut. When the pressure exerted by the blood on the inflow side of the valve exceeds the pressure exerted by the outflow side, the disc begins to open. First, it is forced downstream a very short distance until it firmly contacts the outflow pivot strut 33. Thereafter, the disc pivots about curved end 43 of member 33 while being guided by the central orifice bulge portion 44, which has already been noted, tapers gradually into the final S-shaped free end portion of the guide strut 34. It should be noted that the sole pivot point is the top of member 33 which in cooperation with the guide strut permits movement of the disc 35. Initially, this motion is to pivot the valve disc 35 and ultimately as the valve disc 35 goes through its motion it is also displaced due to the narrowing of the guide strut in a direction outwardly away from the valve base in a downstream direction.

Assuming blood flow were in the outward direction, the disc 35 would move away from valve base 31 the distance equal to the difference between opening 36 and the diameter of tip 45. The maximum ultimate opening of the valve disc 35 is approximately 70°—75° to its original rest position. Inflow pivots 32 also furnish a stop or second surface 41a for the pivoting of disc 35 as the maximum angle of 70°—75° is attained.

The initial pivot point (or chordal pivot axis) 43 is located on the outflow pivot strut 33 and is about 1/4 of the orifice diameter measured from the orifice interior walls. As a result of this location of the initial chordal pivot axis 43, the great majority of the pressure against the inflow side of the disc 35 acts to force it open while the remainder acts to keep the disc 35 closed. Therefore, the disc pivots open almost instantaneously.

During the pivoting open event, the disc 35 exhibits three additional movements. First, the disc 35 moves toward the center or central axis of the orifice. Second, the disc 35 is allowed to move downstream slightly by an amount determined by the design of the free end 45 of the disc guide strut. Finally, the disc 35 is free to rotate at any stage about its own central axis. The disc 35 is angled such that a minimal interference with the flow of blood is provided commensurate with the rapid functioning of the valve in a closing mode.

The closing of the valve is brought about when the pressure on the outflow side is greater than the pressure on the inflow side. When this occurs, blood begins to flow back through the valve and forces the disc 35 into the closed position. The disc 35 is first carried back into the orifice a small distance until restrained by encountering of the circumferential edge of the disc 35 with interior wall portions of the valve annulus and the two inflow pivots. The valve disc 35 then commences to pivot as permitted by the inflow pivots. Throughout the closing event, the disc 35 pivots on the curved line surface of the inflow pivots that project directly into the orifice. When closed, the disc 35 rests in line contact with pivot edges 42 and disc stop surface 39.

## Claims

1. A heart valve for controlling the flow of blood including:

a. a valve base (31) of a generally annular configuration defining a circular opening therethrough, said valve base having an outflow side and an inflow side and an interior wall;

b. inflow pivot members (32) extending inwardly from said wall into said opening along a chordal axis towards one another, said inflow pivot members being spaced towards the inflow side of said valve base;

c. an outflow pivot member (33) extending radially into said opening from said wall and perpendicular to the chordal axis of said inflow pivot members, said outflow member being on the same side of said opening as said chordal axis;

d. a disc guide strut (34) projecting generally inwardly from said wall towards the center of said valve base opening, said strut projecting from said wall from the side of said opening opposite said outflow pivot member (33), said strut (34) defining an S-shaped curve extending diagonally upstream out of said valve base (31) and curving back to pass axially through the center of said opening and then curving diagonally downstream of said valve base (31) to terminate in a free end (45);

e. a valve disc (35) of circular shape having a diameter slightly smaller than said opening, said valve disc defining an axial orifice (36) therethrough of a diameter slightly larger than the diameter of said guide strut (34), said valve disc being mounted in said valve base (31) with said guide strut (34) extending through said orifice (36) and being positioned, when in the closed mode position intermediate said inflow pivot members and said outflow pivot member (33);

f. a valve disc stop shoulder (39) extending inwardly from said wall on the side of said opening opposite said outflow pivot member (33);

g. said inflow pivot members (32) each having a first surface (41a) angled at about 70°—75° with respect to the plane of said valve disc (35) when said disc is in said closed mode, said first surface of said inflow pivot members defining the maximum open mode position of said valve disc; the valve characterized in that:

h. said valve disc stop shoulder (39) has a rounded surface and presents a shoulder edge, said shoulder edge providing a line contact with said disc in said closed mode position of said disc, and in that

i. said inflow pivot members (32) each have a second surface (41) angled at about 15°—20° with respect to the plane of said valve disc (35) when said disc is in said closed mode, said first (41a) and second (41) surfaces of said pivot members meeting to form pivot edges, said pivot edges providing line contacts with said disc in said closed mode position of said disc

whereby the line contacts provided by said valve disc stop shoulder and said pivot edges of said inflow pivot members reduce blood cell damage caused by repeated closings of said valve disc while simultaneously cushioning the closure of the disc.

2. A heart valve in accordance with claim 1, wherein said valve disc stop shoulder (39) is formed by the juncture of said disc guide strut (34) with said wall.

## Revendications

1. Une valvule cardiaque pour commander l'écoulement du sang, comprenant:

a. une monture (31) de valvule de configuration générale annulaire délimitant à travers elle une ouverture circulaire, ladite monture de valvule ayant un côté d'écoulement de sortie et un côté d'écoulement d'entrée et une paroi intérieure;

b. des éléments de pivots d'entrée (32) s'étendant ves l'intérieur depuis ladite paroi dans ladite ouverture suivant l'axe d'une corde l'un vers l'autre, lesdits éléments de pivots d'entrée étant espacés vers le côté d'entrée de ladite monture de la valvule;

c. un élément de pivot de sortie (33) s'étendant radialement dans ladite ouverture à partir de ladite paroi et perpendiculairement à l'axe desdits éléments de pivots d'entrée, formant une corde, ledit élément de sortie étant du même côté de ladite ouverture que ledit axe formant une corde;

d. un support (34) de guidage de disque faisant saillie dans son ensemble vers l'intérieur depuis ladite paroi vers le centre de ladite ouverture de la monture, ledit support faisant saillie sur ladite paroi depuis le côté de ladite ouverture opposé audit élément (33) de pivot de sortie, ledit support (34) formant une courbe en S s'étendant diagonalement en amont à l'extérieur de ladite monture (31) de la valvule et s'incurvant en arrière pour franchir axialement le centre de ladite ouverture et s'incurvant ensuite diagonalement en aval de ladite monture (31) pour se terminer par une extrémité libre (45);

e. un disque (35) de valvule de forme circulaire ayant un diametre légèrement inférieur à celui de ladite ouverture, ce disque délimitant un orifice axial (36) qui le traverse ayant un diamètre légèrement plus grand que le diamètre dudit support (34) de guidage, le disque de valvule étant monté dans ladite monture (31) avec ledit support de guidage (34) s'étendant à travers ledit orifice (36) et étant positionné, lorsqu'il se trouve en position de mode de fermeture entre lesdits éléments de pivots d'entrée et ledit élément de pivot de sortie (33);

f. un épaulement (39) d'arrêt du disque s'étendant vers l'intérieur depuis ladite paroi sur le côté de ladite ouverture opposé audit élément de pivot de sortie (33);

g. lesdits éléments de pivots d'entrée (32) ayant chacun une première surface (41a) inclinée d'environ 70° à 75° par rapport au plan du disque (25) de la valvule lorsque ce disque se trouve dans le mode de fermeture, ladite première surface desdits éléments de pivots d'entrée délimitant la position maximal en mode d'ouverture du disque de la valvule; cette valvule étant caractérisée en ce que:

h. l'épaulement (39) d'arrêt du disque de la valvule comporte une surface arrondie et présente une arête d'épaulement, ladite arête d'épaulement assurant un contact linéaire avec le disque dans ladite position de fermeture du disque, et en ce que:

i. lesdits éléments de pivots d'entrée (32) présentent chacun une seconde surface (41) inclinée d'environ 15° à 20° par rapport au plan du disque (35) de la valvule lorsque ce disque se trouve en mode de fermeture, ladite première (41a) et seconde (41) surfaces desdits éléments de pivots se rencontrant pour former des arêtes de pivotement, lesdits arêtes de pivotement assurant des contacts linéaires avec le disque dans ledit mode de fermeture de ce disque

grâce à quoi les contacts linéaires assurés par ledit épaulement d'arrêt du disque de la valvule et lesdites arêtes de pivotement des éléments de pivots d'entrée réduisent la détérioration des cellules du sang provoquée par des fermetures répétées du disque de la valvule tout en amortissant simultanément la fermeture du disque.

2. Valvule cardiaque suivant la revendication 1, dans laquelle l'épaulement (39) d'arrêt du disque de la valvule est formé par la fonction dudit support (34) de guidage du disque avec ladite paroi.

**Patentansprüche**

1. Herzklappe zum Steuern des Blutstromes mit:

a. einem Klappengrundelement (31) von im wesentlichen ringförmiger Ausbildung, das eine durchgehende kreisförmige Öffnung bildet und eine Ausströmseite und eine Einströmseite sowie eine Innenwand hat;

b. Einströmgelenkteilen (32), die von der Wand entlang einer Sehnenachse in die Öffnung hinein und aufeinanderzu reichen und die in Richtung auf die Einströmseite des Klappengrundelements in Abstand liegen;

c. einem Ausströmgelenkteil (33), das von der Wand radial in die Öffnung hinein und senkrecht zu der Sehnenachse der Einströmgelenkteile verläuft und das sich auf der gleichen Seite der Öffnung wie die Sehnenachse befindet;

d. einer Klappenführungsstrebe (34), die von der Wand im wesentlichen einwärts in Richtung auf die Mitte der Klappengrundelementöffnung vorsteht, von der Wand an der dem Ausströmgelenkteil (33) gegenüberliegenden Seite der Öffnung ausgeht und eine S-förmige Kurve bestimmt, die sich diagonal stromaufwärts aus dem Klappengrundelement (31) herauserstreckt und zurückgebogen ist, um durch die Mitte der Öffnung axial hindurchzureichen, und die dann diagonal stromabwärts von dem Klappengrundelement (31) gebogen ist, um in einem freien Ende (45) auszulaufen;

e. einer Ventilklappe (35) von kreisförmiger Gestalt mit einem etwas kleineren Durchmesser als die Öffnung, wobei die Ventilklappe eine durchgehende axiale Öffnung (36) mit einem etwas größeren Durchmesser als dem Durchmesser der Führungsstrebe (34) bildet und in dem Klappengrundelement (31) derart gelagert ist, daß die Führungsstrebe (34) durch die Öffnung (36) hindurchreicht, und wobei die Ventilklappe in der Schließstellung zwischen den Einströmgelenkteilen und dem Ausströmgelenkteil (33) liegt;

f. einer Ventilklappen-Anschlagschulter (39), die von der Wand an der dem Ausströmgelenkteil (33) gegenüberliegenden Seite der Öffnung ausgeht;

g. wobei die Einströmgelenkteile (32) jeweils eine erste Oberfläche (41a) aufweisen, die in einem Winkel von etwa 70° bis 75° mit Bezug auf die Ebene der Ventilklappe (35) steht, wenn sich die Ventilklappe in der Schließstellung befindet, und wobei die erste Oberfläche der Einströmgelenkteile die maximale Offenstellung der Ventilklappe bestimmt; dadurch gekennzeichnet, daß:

h. die Ventilklappen-Anschlagschulter (39) eine abgerundete Oberfläche hat und eine Schulterkante bildet, die für einen Linienkontakt mit der Ventilklappe in der Schließstellung der Ventilklappe sorgt, und daß

i. die Einströmgelenkteile (32) jeweils eine zweite Oberfläche (41) aufweisen, die in einem Winkel von etwa 15° bis 20° mit Bezug auf die Ebene der Ventilklappe (35) steht, wenn sich die Ventilklappe in der Schließstellung befindet, wobei sich die erste Oberfläche (41a) und die zweite Oberfläche (41) der Gelenkteile unter Bildung von Gelenkkanten treffen, die für Linienkontakte mit der Ventilklappe in der Schließstellung der Ventilklappe sorgen, wodurch die von der Ventilklappen-Anschlagschulter und den Gelenkkanten der Einströmgelenkteile bewirkten Linienkontakte Blutzellenschäden verursacht durch wiederholtes Schließen der Ventilklappe vermindern, während sie gleichzeitig das Schließen der Ventilklappe abfedern.

2. Herzklappe nach Anspruch 1, wobei die Ventilklappen-Anschlagschulter (39) von der Verbindung der Klappenführungsstrebe (34) mit der Wand gebildet ist.

PRIOR ART

FIG. 1a

FIG. 2a

PRIOR ART

FIG. 1b

FIG. 2b

PRIOR ART

FIG. 1c

FIG. 2c